# EUROPEAN PATENT SPECIFICATION

(11) **EP 2 056 891 B1**
(45) Date of publication and mention of the grant of the patent: **11.10.2017**
(21) Application number: 07800457.9
(22) Date of filing: 17.08.2007
(51) Int. Cl.: A61L 15/60, A61L 15/24, A61L 15/44, A61L 15/58, A61L 15/42

(54) **AN INTERPENETRATING NETWORK OF PVA HYDROGEL COOL-COMPRESSION BANDAGE**
INTERPENETRIERENDES NETZ AUS PVA-HYDROGEL-KÜHL/KOMPRESSIONSVERBAND
RÉSEAU D'INTERPÉNÉTRATION D'HYDROGEL À BASE DE PVA ET PANSEMENT COMPRESSIF FROID

(30) Priority: 17.08.2006 US 838204 P
(43) Date of publication of application: 13.05.2009
(73) Proprietor: ATS Biotech, Inc., Oakville, ON L6L 3B7 (CA)
(72) Inventor: HARTWELL, Ryan, Ottawa, Ontario K1G 0E8 (CA)
(74) Representative: FRKelly
(86) International application number: PCT/CA2007/001429
(87) International publication number: WO 2008/019498

(56) References cited:
- WO-A1-02/087645
- WO-A1-03/057267
- WO-A2-02/055113
- US-A- 3 998 215
- US-A- 4 552 138
- US-A- 4 699 146
- US-A- 5 260 066
- US-A- 5 527 271
- US-A1- 2005 080 368

## Description

### FIELD OF INVENTION

The present invention relates to a tape or bandage that provides cool-compression, as well as natural analgesics and anti-inflammatories for the relief of pain and inflammation as a result of injury or disease.

### BACKGROUND OF THE INVENTION

The therapeutic value of simultaneously applying cold and compression to an injury is well-established. The Merck Manual of Medical Information states that the immediate treatment of certain types of injuries such as sprains requires Rest, Ice, Compression and Elevation (RICE). Although Rest and Elevation can be attained without any special equipment, the Ice and Compression components require the use of devices.

Elasticized wraps can be used to apply compression to an injured area. Specialized bandages that are adapted to various body parts, such as shoulders, wrists, knees, etc. are also available. These bandages provide compression but not cooling.

While a simple ice bag can be used to apply cooling, it is not very convenient as the ice melts. Several other attempts have been made to obtain a cooling bandage. For example, a cooling effect can be provided by a pouch that contains separate chambers containing different chemical components. When the chambers are disrupted and the different chemicals come into contact with each other there is an endothermic reaction which provides a cooling sensation. This type of device is not re-usable and it does not provide concomitant compression. A system that circulates cold water within a bandage is available, but this system is bulky and immobile.

DURA KOLD sells reusable ice wraps that comprise pillows of purified water with food-grade freezing agents which are sewn into wraps that can be applied using Velcro straps. Dura Kold products are designed to be applied to a variety of locations and deliver sufficient cold therapy for post-operative application, bumps, bruises, strains, sprains, etc.

A 3K Cryotherapy Compression Bandage from Silipos that includes a U-shaped gel filled cooling element in a sleeve that wraps around an injured joint is also available.

Other cooling bandages are in development. United States Patent Application 2004/0153040 describes a multi-layered polyurethane foam dressing that comprises an outer layer of a hydrogel formulated from polyurethane or an adhesive elastomeric material; a hydrophilic polyurethane foam layer and a non-adherent surface contacting cooling layer of a polyurethane hydrogel.

United States Patent application 2005/0080368 discloses a bandage with cooling capabilities that includes bandage support members and a cold pack between them. US 4,552,138 discloses a dressing material of at least one layer of a polymeric, hydrophilic gel and, where relevant, one or more layers of a carrier material as an intermediate and/or covering layer. The gel consists of 40-50% by weight of a polyvinyl alcohol which is crosslinked and acetalized with formaldehyde and is water-insoluble to the extent of at least 90%, 15-30% by weight of water, 15-45% by weight of one or more polyhydric alcohols with 2-6 C atoms and, where relevant, small amounts of additives and/or auxiliaries.

Thus a wide variety of systems have been advanced for the application of cold and compression. However, none of the known devices has addressed the clearly established need for maximizing compression and cold in areas where needed. Thus, there was a need for an improved system that is re-usable, economical and flexible for use on different body parts. Furthermore, topically applied ointments and creams that contain natural extracts or synthetic versions thereof, have long been used to alleviate pain and reduce inflammation. However, the practice of incorporating these compounds into a dressing has posed certain technical difficulties and has not previously been described.

### SUMMARY OF THE INVENTION

The present invention relates to a cool compression bandage for the treatment of pain and swelling.

The present invention is directed to a method of preparing a compression bandage comprising:
i) a fibrous matrix of a pulp based, airlaid, non-woven or absorbent open-cell elastomer network of greater than or equal to 70% void volume; and
ii) an interpenetrating network of PVA disposed within the elastomer network, wherein said method comprises;
   (a) preparing a PVA gelling solution;
   (b) immersing the fibrous matrix in the gelling solution;
   (c) adding a crosslinking solution comprising tetra-borohydrate, Borax or Boron decahydrate; and
   (d) removing excess water.
The present invention is also directed to a compression bandage obtainable by the the method above.

In a preferred embodiment, the PVA network comprises a plasticizer selected from the group consisting of: propylene glycol, polypropylene glycol, ethylene glycol, diethyline glycol and tri-ethylene glycol.

In another embodiment, eicosapentaenoic acid (omega-3) is used in combination with a glycol or glycerol agent to plasticize, as well as to act as an antioxidant and as an anti-inflammatory. Alpha-tocopherol and its acetate form can also be included within this composition.

In another preferred embodiment, the PVA network further comprises an agent selected from the group consisting of Aloe Vera, paraben, Arnica, BHT, alphatocopherol, sodium diacetate, cineol, menthol, Noni extract, camphor, white fir oil, imortelle, betulin alba terpinoids, lavender oil derivatives, alpha-terpinol, niacin, zinc ascorbate dehydrate, zinc oxide, methyl-salicylate, and zinc sialic-acid. Other zinc salts may be included.

In another aspect of the invention, a method of formulating a cooling bandage is provided. The method comprises: dissolving PVA in distilled-deionized water with heat and stirring until the PVA is completely dissolved; adding a plasticizer and stirring; cooling the solution to room temperature; and adding an Aloe Vera composition to the solution.

Various types of substrates can be used as the support structure for the bandage/dressing. For example, the substrate for the bandage can include a pulp-based non-woven, biodegradable synthetic polymer such as polyamide foams or elastomeric materials. Natural fibers such as cellulose, alginate, hemp, cotton and bamboo can also be used. In one preferred embodiment hemp fibers are pre-treated with a plasticizer or surfactant.

In another aspect of the invention, the dressing comprises a patch or compress that can be applied to promote cooling. For example, a patch can be applied to promote cooling of an inflamed area, such as a vaccination site. Alternatively, the dressing may be applied in a manner similar to a compress used to reduce a fever.

This summary of the invention does not necessarily describe all features of the invention.

### BRIEF DESCRIPTION OF THE DRAWINGS

These and other features of the invention will become more apparent from the following description in which reference is made to the appended drawings wherein:
FIGURE 1 shows a roll of tape bandage according to the invention.
FIGURE 2 illustrates one embodiment of a use of the invention.

### DETAILED DESCRIPTION

The following description is of a preferred embodiment.

The present invention provides a cool compression bandage designed to provide relief from pain and swelling as a result of injury and disease. The term "bandage" as used herein encompasses wrappable lengths, patches, plasters, compresses and the like.

The bandage comprises an interpenetrating network (IPN) of polyvinyl alcohol (PVA) and glycerol or propylene glycol, poly propylene glycol, triethylene glycol or glycerin-like derivatives cross linked via hydrogen bonding and ionic interactions with tetra-borohydrate, Borax or Boron decahydrate.

The osmotic balance may be adjusted using CaCl₂ or another salt, preferably, not necessarily, in UV sterilized nano-purified distilled water.

The network forms an isotropic system that, when swollen, will retain up to 500% of its dry weight in water or other solvent. This network is preferably created within a porous structure, such as an open cell toluene diisocyanate polyurethane foam or an absorbent open cell elastomer foam network, such as an airlaid non-woven. The network forms an isotropic system that, when swollen, will retain up to 500% of its dry weight in water or other solvent.

In a preferred embodiment, additional therapeutic agents are included. For example, Aloe Vera whole leaf extract (A. Vera) and/or Arnica is preferably added to the PVA gel solution prior to crosslinking thereby entrapping the natural product for later diffusion applications. Other agents, such as 1, 8 cineol and/or camphor, mallelucca oil, DL- menthol, White fir oil (beta-caryophyllene, limonene, alpha pinene), Betulin Alba terpene oils (betulinic acid), Immortelle oil (gamma-curcumene, neryl acetate, italidiones), Lavender oil (linalool, linalyl acetate), zinc acetate, zinc oxide, zinc ascorbate dehydrate, Niacin (B3), noni fruit extract and/or alpha-terpineol are typically added to the wrap in the cross linking stage or one step prior to, such that the natural product is easily and rapidly released from the bandage upon application. Other optional additives include antioxidants, anti-fungal and antibacterial agents are also added to the bandage to enhance its efficacy and durability. Standard commercial agents such as methyl paraben, propyl paraben, BHT and/or alpha-tocopherol and sodium diacetate may be used. Preferred use of the bandage is for treatment of pain and/or swelling. It is clearly apparent that various types of therapeutic agents in addition to those specifically mentioned can be included. Therapeutic agents may be included at various stages in the process.

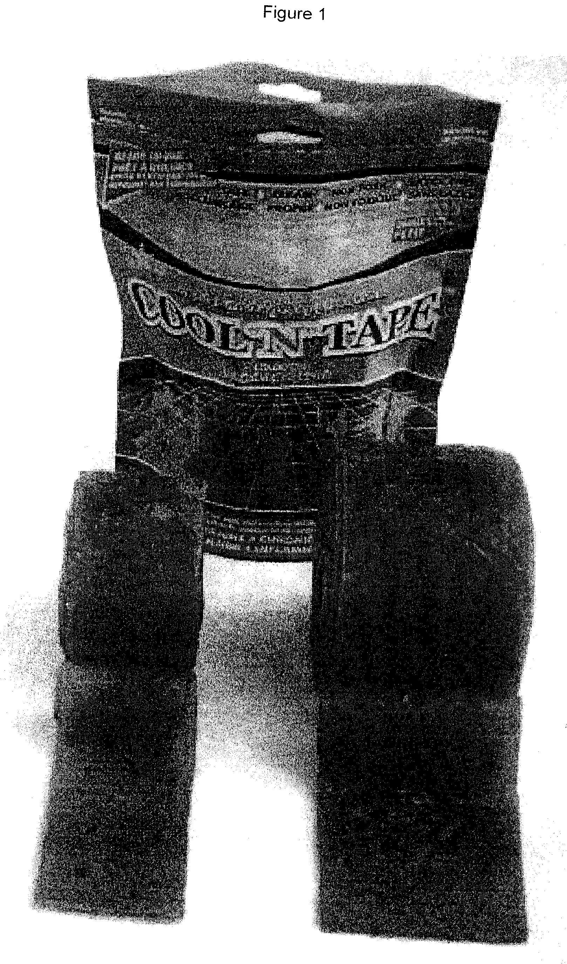

In one preferred embodiment, the bandage is provided in 1,22-1,83 m (4-6 foot) lengths, but can be produced longer or shorter. Figure 1 illustrates a roll of bandage that can be used as is or cut into smaller lengths. While certain aspects of this description have focused on the application of a bandage for compression, the bandage may also be provided as a patch or shorter length to provide cooling of inflamed tissue or to treat fever. The width may vary. It is self-adherent and non-adherent to skin. During its first application, the bandage remains cold (18-22°C) for up to an hour and reduces skin surface temperature an average of 10°C during this period (normally 37-38°C). For reuse, the bandage can be re-wetted with a small amount of cold water, rewound, sealed and placed in the fridge until the next use. Subsequent applications (up to five times) have been demonstrated to be effective for half hour durations. Prior to the first time application, the bandage can be stored at or above room temperature if air sealed. The bandage can withstand freezing/thawing and will not freeze to complete solid above temperatures of - 10°C due to the glycerol or other freezing depressive agents in the bandage. It is recommended that a bandage be thawed to 5°C before use. When the bandage is placed in environments between 5°C and 30°C it remains cool at or below 22°C and has a smooth, moist texture that does not leave a residue. The bandage remains cool and feels moist because of its tendency to slowly release water vapor. As water is released from the bandage, the additional agents discussed above are released by simple diffusion onto the skin surface. These agents are released at the surface as the initial water molecules change phase. Natural agents such as DL-menthol create an increased cooling effect by binding to sensory receptors in the dermal layer and increasing the porosity of the oil/water barrier of the stratum corneum. Zinc mediates both antioxidant and analgesic functions by binding to cysteine rich proteins and blocking NMDA receptors. Cineol acts as an immune modulator of TNF-a, natural cooling agent (evaporative tendency of the ether), as stimulant (smell) and as a natural anti-microbial. Omega-3 (EPA) functions as a membrane and dermal replenishment, as a natural inhibitor of COX-1 (as a leukotrienes) and analgesic. Natural products and essential oils to reduce pain, inflammation and bruising as a result of injury or disease may be included. Agents such as 1, 8 cineol, and DL-menthol are incorporated at or near the surface of the IPN network to be more readily available and to have a greater therapeutic effect. Aloe Vera WLE gel, which is a gel solution composed of amino-glycosides, peptidoglycans, cathecins and glycopeptide like derivatives, produces a thickening effect on the initial PVA gel solution. This contributes to more rapid ionic crosslinking and reduces the rate of water evaporation thereby prolonging the stability of the bandage. The natural products found within the A. Vera gel (predominantly Eugeol and cytchrome analogues) can diffuse in small amounts through the pore structure of the gel onto the skin for therapeutic purposes.

In one aspect of the invention, a method of preparing a cold compression bandage is also provided. Synthesis of the IPN network begins with immersion of an open cell foam into a PVA gelling solution at room temperature. In a preferred embodiment of the method, the foam is heated slightly to about 1/3 of its melting point to relax the bonds within the foam network. The foam is then passed through a series of pinch rollers at a sufficient depth to mechanically remove the air from the foam. The air is guided to the surface with a weir. The foam typically requires a minimum duration of 1 minute within the PVA gel solution to ensure complete absorption. Once the foam has completely absorbed the PVA gel, it is optionally passed through an essential oils bath, which will saturate the surface structure of the gel with natural agents. Alternatively, the essential oils may be placed in solution (although mainly suspended within the surface layers) with the tetra-borohydrate (borax) crosslinking agent. To ensure complete hydrogel formation the foam preferably remains within the cross linking solution for a minimal duration of 3 minutes traveling 90% of the time within the solution and 10% of the time out of the solution for degassing. The hydrogel bandage is passed under a slanted ambient (cool) air knife that is angled to remove excess water, allowing the cross linking to complete. After the excess water is removed, the bandage is rolled, cut and packaged. For best results the finished product is packaged and refrigerated at 5°C for 24hr to completely cure the gel network. It has been noted that a freeze/thaw cycle will produce a much more rubber-like product that will not evaporate as quickly as one prepared without any refrigeration or freezing.

In another method, the PVA gelling solution, as well as the NHP borax solution is formulated in-line. The heated solutions, at approximately 60°C, are directed to an injection mixer where they intersect and mix proportionally in a 4:1 - 3:1 ratio (PVA solution:Borax/NHP solution) at a controlled temperature of about 50°C and a pH of about 7.5. The mixing of the two solutions generates a very viscous gel at room temperature. However, at higher temperatures, it will remain as a viscous resin. Below 50°C the PVA solution will begin to gellate, forming colloidal bodies within the mixture. The gelled mixture is then forced through porous nozzles and injected into a compressed hydrophilic poly-urethane foam. The gel resin (mixture) impregnates the foam and when the foam expands, it is entrapped within, at which point the change in temperature will have allowed the gel resin to solidify into a complete gel state. The gelation state can occur long before it is qualitatively evident and thus temperature and pH control are essential. With reference to pH, the pH is of the gelling region is found to be between pH 7 - 8, with an initial change in viscosity at pH 6. Premature crosslinking will however cause colloid formation and increased viscosity, thus mixing is best achieved in the nozzle. Crosslinking of PVA through hydrogen bonding and ionic interactions is irreversible and thus, once gone to completion, cannot be reduced by heat or pH. However, it has been found that at up to pH 8, the gel may be reversibly titrated with equimolar amounts of base and acid.

In another aspect of the invention, a method of preparing the IPN begins with the immersion of a pulp-based material into heated (40°C) PVA mix. Once fully saturated (<2min) the material is removed from the bin and excess PVA is removed. Ideally the amount of PVA uptake is equivalent to the volume of the material. The PVA impregnated material is then spray-coated with an emulsion containing a hydrophobic essential oil and/or terpene and or/polyphenol and/or flavonoid and/or any other oil such as EPA (omega-3) that is not incorporated within the PVA mix. The material is then submerged into a boron based (borax) crosslinking solution (pH= 8, 21°C) containing dl-menthol, cineole, methyl salicylate or other such compounds previously mentioned. Crosslinking typically requires 2-3minutes depending upon the size of material, the amount of PVA and whether the hydrophobic spray was employed. After immersion, a hand held ambient air blower is used to remove excess crosslinker solution. Finally, the material is rolled onto PS tubes (or a tube of similar nature). The final weight of the product is approximately 400-500x or greater than the weight of the starting material.

A higher throughput method of preparing synthesis of the IPN begins with immersion of the pulp-based material into a PVA gelling solution at room temperature. For best absorption, the PVA is heated to 40°C reduce viscosity and hydrogen bonding. The material is pre-dipped within a trough of PVA, then passed through a curtain of PVA mix immediately followed by a series of pinch rollers that sufficiently and mechanically saturate the material. The entire process can be completed within less than 5 seconds. Once the foam has completely absorbed the PVA gel, it may be sprayed with additional (yet non-soluble essential oils, terpenes, ethers, polyphenols, flavonoids and or any other oil that is not incorporated within the PVA mix. Alternatively, the essential oils may be (although mainly emulsified) within the crosslinker (borax or derivative described above) crosslinking agent and the material may pass through dip tanks crosslinking therein. To ensure complete hydrogel formation the foam preferably remains within the cross linking solution for a minimal duration of 3 minutes traveling 90% of the time within the solution and 10% of the time out of the solution for degassing. The hydrogel bandage is passed under a slanted ambient (cool 18°C) air knife that is angled to remove excess water, allowing the cross linking to complete. After the excess water is removed, the bandage is rolled, cut and packaged. For best results the finished product is packaged and refrigerated at 5°C for 24hr to completely cure the gel network. It has been noted that a freeze/thaw cycle will produce a much more rubber-like product that will not evaporate as quickly as one prepared without any refrigeration or freezing.

To employ an even higher throughput method, the material once saturated with both PVA mix and oils or just PVA is then passed through a spray box (travel time <45sec). The spray box uses a non-aspirating nozzle that covers the material with the borax crosslinker within a cone spray.

The precise formulation of the bandage affects the properties of the final product. The percent composition of the PVA gelling solution and the natural product solution and/or the cross linking solution are the basis for the composition of the final bandage, yet they are only maximal representations of the actual composition found within the bandages. Therefore, the amount of natural ingredients actually incorporated into the bandage may be less than in the solutions. Thus, the description of the compositions described below are those of the starting solutions.

The PVA gelling solution typically comprises about 7-10%w/v PVA, preferably 7%w/v, 3 parts 86% hydrolyzed low molecular weight and 1 part 99% hydrolyzed medium molecular weight. The gelling solution further comprises 2-5%w/v preferably 5%w/v, glycerol or propylene glycol, polypropylene glycol, ethylene glycol, diethylene glycol, triethylene glycol or glycerin. About 2-5% w/v EPA and about 2-5% w/v Arnica are preferably included. About 2-8%w/v preferably 5%w/v Aloe Vera whole leaf extract gel is included. 2-5%w/v Arnica and less then or equal to 1%w/v of methyl paraben, propyl paraben, BHT and/or alpha-tocopherol, and sodium diacetate is optionally included. The natural product solution contains, with some degree of variability based on the desired properties of the bandage, 1-7%w/v 1,8 cineol, and/or 1-5%w/v DL-menthol, 5%w/v Noni extract, 1-5%w/v camphor, 1-5%w/v White Fir oil derivatives, 1-5%w/v Immortelle derivatives, 5% zinc ascorbate dihydrate or other zinc derivatives, 2-7%w/v Betulin Alba terpinoids, 1-5% methyl salicylate, 2%w/v Lavender oil derivatives, and/or 2%w/v alpha-terpineol, 2% niacin. The cross linking solution contains 7-10%w/v borax, preferably 10%w/v and 7-10%w/v glycerol and one of its said derivatives, preferably 7%w/v. Also included are 1% of each parabens, alpha-tocopherol/acetate and sodium diacetate. However, if the crosslinking solution contains the natural products, the proportions are about 4-5%w/v 1, 8 cineol, 1-4%w/v DL-menthol, 2-4%w/v camphor, 2-4%w/v White Fir oil derivatives, 2-4%w/v Immortelle derivatives, 2-4%w/v Betulin Alba terpinoids, 2-4%w/v Noni extract, 2-4%w/v Lavender oil derivatives, 2%w/v alpha-terpineol, 1-2%methyl salicylate, 5% zinc ascorbate dihydrate, 2%niacin.

The solutions described above may be combined in a number of ways, that typically involve a period of equilibrium of about 1-2 hours of large batch or shorter periods of inline mixing at 21°C as described previously. The formulation of the cross linking solution can be varied depending on whether the bandage requires a long lasting, high concentration of natural products or whether the natural products are to be lesser in concentration and for a shorter duration of release. If a long-lasting supply is needed, a stand alone natural product solution is best formulated if dissolved in isopropyl, ethylene glycol, propylene glycol, tri or di-ethylene glycol, preferably isopropyl, and mixed part by part until homogeneous at 30°C. Alternatively, if the desired product is to be of 'regular strength' or short duration/low natural product dose, then the natural products may be added to the cross linking solution in a part by part addition until homogenous in a water solvent starting with borax as the initial solute.

The method involves one hour of mixing at a temperature greater than 30°C. This formulation will produce an optimal crosslinking solution with a pH of 8.1-8.8. Upon mixing of the solutions into a pre-cryogel, the PVA saturated foam forms a colloidal barrier around the foam within the crosslinking bath. Over the 3min duration the gel becomes rigid and distinct and the weight of the saturated PVA foam increases nearly 500% of its PVA gel-saturated weight. To increase this rigidity, it has been found that the addition of tri-ethylene glycol, with respect to the bulk of plasticizers will reduce the mobility of PVA chains and the rate of water evaporation within the gels the most and consequently obtain the smallest pore size within the IPN.

The therapeutic effect of the device is achieved in three ways. Firstly, moderate cooling of an injured, inflamed, bruised tissue or found source of analgesia reduces the symptoms and biochemical responses that occur within such site by reducing the size of microvasculature, and secondly, by providing compression to limit the extent of edema (or extracellular fluid perfusion) and thirdly, the moderate addition of natural therapeutic agents assists in the relief of analgesia and reduction of inflammation to promote the healing of the affected tissue.

The device has several advantages over other bandages in terms of providing relief from pain and inflammation. The device of the invention provides cooling and compression in an integrated system. The tape can be cut in various sizes depending on the need and it can be re-used. DL-menthol and 1, 8-cineol increase permeability of the stratum corneum and increase sensitivity of the local cold receptors located within the epidermis and dermal layers. The IPN gel/network is hydrophilic and absorbent thus it leaves no residue behind once removed. The IPN gel/network is re-useable with respect to its instructions. The device may be used for durations up to an hour without damaging peripheral tissue layers by the application of 'cold'. The device upon first one-hour use may be stored at room temperature or slightly above and well below (-20°C - 45°C). The IPN gel/network permits the diffusion of water once saturated and any other natural component within.

The gel is non-toxic and biodegradable, yet it can withstand moderate photoemission (degradation). The IPN gel/foam network remains at or near a constant temperature of 18°C for durations up to an hour when exposed to an open environment. The IPN gel/substrate network is an elastomer, with a given tensile strength of approximately 200kPa, which will tear if an injury is wrapped too tight. The gel itself has a tensile strength of 85kPa. The IPN gel/foam network is self-adherent and will not stick to skin. The IPN gel/foam network is breathable- able to provide oxygen exchange with covered tissue areas. The IPN gel/foam network contains in an appreciable amount some form or combination of (a) natural product(s) which include any of the following and derivatives of such:
a. Aloe Vera, antioxidant, anti-microbial, anti-inflammatory, chelators, cytochromes, peptidoglycans and glycoproteins;
b. Arnica- used in the treatment of bruising and muscular skeletal rehabilitation;
c. 1, 8 cineole- most active ingredient in eucalypt oil, anti-inflammatory, expectorant, anti-analgesic, increases hydrophobic barrier, membrane fluidity;
d. DL-menthol-anti-analgesic, binds to sensory receptors;
e. Camphor- Blood flow stimulating agent;
f. White Fir extract (Limonene, alpha-pinene, beta-caryophyllene) - sooths stiff and soar muscles and stimulates blood flow (all components are aromatic and may pose some irritability);
g. Noni extract- anti-oxidant, anti-inflammatory, anti-tumor, epithelial regeneration;
h. Immortelle - anti-inflammatory and wound healing-expectorant/decongestant;
i. Lavender -anti-inflammatory, anti-analgesic and aids in bruise healing. In addition to lavender MYRTLE may also be added for the same reasons (this contains very similar components);
j. Betulin Alba- Betulinic Acid derived terpinoids for their anti-inflammatory effect and systemic anti-analgesic/benzodiazepine like effect;
k. Zinc ascorbate or other zinc compounds (salts) - antioxidant, anti-inflammatory, proliferative (repair) agent, immune stimulant, tissue regeneration, analgesic and gene expression; and
l. Niacin (B3) Nicatinomide co-factor for NADPH- antioxidant, proliferative (repair) agent, metabolic agent.

In a preferred embodiment, the IPN gel/foam network contains at least one of the following agents and/or:
a. Aloe Vera, antioxidant, anti-microbial, anti-inflammatory, chelator, cytochromes, peptidoglycans and glycoproteins;
b. Arnica- used in the treatment of bruising and muscular skeletal rehabilitation;
c. 1, 8 cineol- most active ingredient in eucalypt oil, anti-inflammatory, expectorant, analgesic, increases hydrophobic barrier, membrane fluidity;
d. DL-menthol- analgesic, binds to sensory receptors;
e. Camphor- Blood flow stimulating agent;
f. White Fir extract (Limonene, alpha-pinene, beta-caryophyllene) - sooths stiff and soar muscles and stimulates blood flow (all components are aromatic and may pose some irritability);
g. Noni extract- anti-oxidant, anti-inflammatory, anti-tumor, epithelial regeneration;
h. Immortelle - anti-inflammatory and wound healing-expectorant/decongestant;
i. Lavender -anti-inflammatory, analgesic and aids in bruise healing. In addition to lavender MYRTLE may also be added for the same reasons;
j. Betulin Alba- Betulinic Acid derived terpinoids for their anti-inflammatory effect and systemic analgesic/benzodiazepine like effect;
k. Zinc ascorbate or other zinc compounds (salts) - antioxidant, anti-inflammatory, proliferative (repair) agent, immune stimulant, tissue regeneration, analgesic and gene expression; and
l. Niacin (B3) Nicatinomide co-factor for NADPH- antioxidant, proliferative (repair) agent, metabolic agent

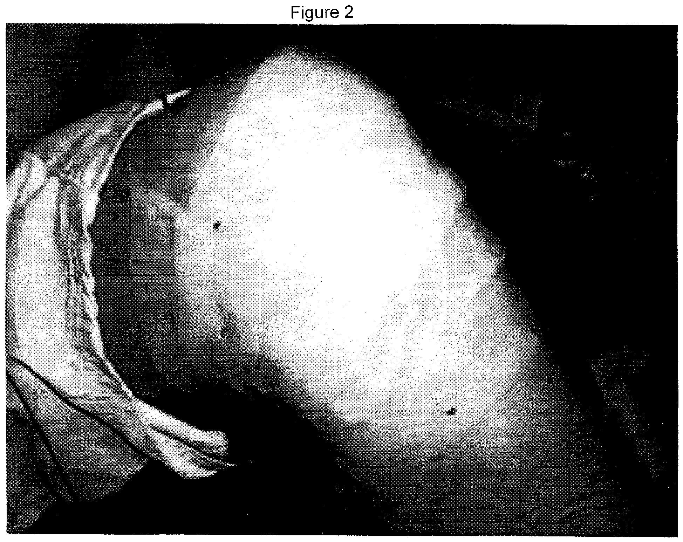

In one aspect, the invention provides a compression bandage that also provides cooling. Figure 2 illustrates one example of how the bandage can be wrapped around an inflamed joint. It is clearly apparent that the bandage material can be configured in various shapes depending on the application.

In another aspect, the invention provides a patch-like dressing.

The invention also provides a polymer matrix for use in the bandage and methods of making and using the bandage. The invention addresses the need for an improved treatment modality for pain and inflammation and promotes healing of injured tissue.

The present invention is useful for first aid treatment of sprain, strains or fractures. It can also be used to treat headaches, migraines, joint pain, inflammation, fever, and other hyperalgesia. Other uses include as a postoperative pain reducer, as an immobilizer for joints and limbs, for relief of tooth aches, for treatment of inflammatory joint and skin diseases and for soft tissue bruising.

In other aspects, bandage strips can be used to relieve lower back pain and rectangular segments can be coated with non-toxic adhesives for use as dressings, patches or plasters to provide topical analgesic

The above disclosure generally describes the present invention. It is believed that one of ordinary skill in the art can, using the preceding description, make and use the compositions and practice the methods of the present invention. A more complete understanding can be obtained by reference to the following specific examples. These examples are described solely to illustrate preferred embodiments of the present invention and are not intended to limit the scope of the invention.

### EXAMPLES

Although specific terms have been used in these examples, such terms are intended in a descriptive sense and not for purposes of limitation. Methods of molecular biology, biochemistry and chemistry referred to but not explicitly described in the disclosure and these examples are reported in the scientific literature and are well known to those skilled in the art.

### Example 1. PVA formulation

630mL of UV and nanopure distilled water is heated to 60°C for 10min, then 0.378g of Propyl paraben, 0.378g of methylparaben, 0.378g of sodium diacetate are added and stirred until dissolved. Then 17.5g of PVA 88% hydrolyzed and low molecular weight is added and stirred until equally dispersed; 52.5g of PVA 99% hydrolysis and medium molecular weight is stirred-in until PVA mix is homogeneous (clear) - pH 5.0. Once homogenous the solution is allowed to cool to 21°C whereat, glycerol 30mL plus 10mL of EPA and 0.07g of a-tocopherol and 1 mL of tocopherol acetate are added followed by 20mL of Aloe Vera gel. The solution is then subsequently stirred for 1-2 hours (pH= 5.0-5.2). Inline mixing can be used rather than conventional batch methods and thus, speeds up the process.

### Example 2. Crosslinker formulation

630mL of UV treated nanopure distilled water, heated to 30°C and therein 0.35g of propylparaben, 0.35g of methyl paraben and 0.225g of CaCl₂ are dissolved. Once dissolved, borax 70g (sodium boro-decahydrate) is dissolved, followed by the addition of 35mL glycerol, 1,8 cineole (1.4mL) and dl-menthol (0.7mL) pH=8.

### Example 3. Preparation of a Bandage

A PVA solution is formulated by dissolving 7%w/v PVA (3:1, 86%:99% hydrolyzed) in distilled-deionized water. The solution is heated, while stirring at 85°C for over one hour until the PVA becomes completely dissolved, after which time 2% w/v glycerol or one of the plasticizers is added to mixture and stirred for an additional 15min. Once the solution is homogenous and transparent (absence of colloid formation) it is cooled to room temperature (stirring continuously). At room temperature (21 °C), A. Vera WLE gel is gradually stirred into the solution at 5% w/v. Once the PVA gel solution is complete it should produce a pH reading of 4.8-5.5. The formulation of the cross linking solution can be varied depending on whether the bandage requires a long lasting, high concentration of natural products or whether the natural products are to be lesser in concentration and for a shorter duration of release. If a long-lasting supply is needed, a stand alone natural product solution is best formulated if dissolved in isopropyl, ethylene glycol, propylene glycol, tri or di-ethylene glycol, preferably isopropyl, and mixed part by part until homogeneous at 30°C. Alternatively, if the desired product is to be of 'regular strength' or short duration/low natural product dose, then the natural products may be added to the cross linking solution in a part by part addition until homogenous in a water solvent starting with borax as the initial solute. The method involves one hour of mixing at a temperature greater than 30°C. This formulation will produce an optimal crosslinking solution with a pH of 8.1-8.8. Upon mixing of the solutions into a pre-cryogel, the PVA saturated foam forms a colloidal barrier around the foam within the crosslinking bath. Over the 3min duration the gel becomes rigid and distinct and the weight of the saturated PVA foam increases nearly 500% of its PVA gel-saturated weight. To increase this rigidity it has been found that the addition of tri-ethylene glycol, with respect to the bulk of plasticizers will reduce the mobility of PVA chains within the gels the most and consequently obtain the smallest pore size within the IPN.

## Claims

1. A method of preparing a compression bandage comprising:
i) a fibrous matrix of a pulp based, airlaid, non-woven or absorbent open-cell elastomer network of greater than or equal to 70% void volume; and
ii) an interpenetrating network of PVA disposed within the elastomer network, wherein said method comprises;
(a) preparing a PVA gelling solution;
(b) immersing the fibrous matrix in the gelling solution,
(c) adding a crosslinking solution comprising tetra-borohydrate, Borax or Boron decahydrate; and
(d) removing excess water.

2. A method according to claim 1, wherein the gelling solution is at 35-45°C in step (b).

3. A method according to claim 1, wherein the crosslinking solution is at 19 to 23°C in step (c).

4. A method according to claim 1, further comprising the step of packaging and then cooling the bandage to about 5°C.

5. A method according to claim 1, wherein the PVA gelling solution comprises 6 to 10% PVA w/v, 1 - 10% of a plasticizer w/v, 2 to 8% Aloe Vera w/v and 1 - 7% of an agent selected from the group consisting of Aloe Vera, paraben, Arnica, BHT, alpha-tocopherol, sodium diacetate, cineole, menthol, Noni extract, camphor, white fir oil, imortelle, betulin alba terpinoids, lavender oil derivatives, cinnamon oil, zinc ascorbate dihydrate or zinc derivatives, niacin, alpha-terpinol, and combinations thereof.

6. A method according to claim 5, wherein the plasticizer is selected from the group consisting of propylene glycol, polypropylene glycol, ethylene glycol, diethylene glycol, tri-ethylene glycol and mixtures thereof.

7. A method according to claim 1, further comprising adding an additive selected from antioxidants, anti-fungal and antibacterial agents.

8. A method according to claim 1, further comprising adding a therapeutic agent.

9. A compression bandage obtainable by a method as defined in any one of the preceding claims.

## Patentansprüche

1. Verfahren zur Herstellung eines Kompressionsverbands, der Folgendes umfasst:
i) eine Fasermatrix eines Pulpe-basierten, luftgelegten Vliesstoff- oder absorbierenden offenzelligen Elastomernetzwerks von größer als oder gleich 70 % Hohlraumvolumen; und
ii) ein interpenetrierendes Netzwerk von PVA, das innerhalb des Elastomernetzwerks angeordnet ist, wobei das Verfahren Folgendes umfasst:
(a) Herstellen einer PVA-Gelierlösung;
(b) Eintauchen der Fasermatrix in die Gelierlösung;
(c) Zugeben einer Vernetzungslösung, die Tetraborhydrat, Borax oder Bordecahydrat umfasst; und
(d) Entfernen von überschüssigem Wasser.

2. Verfahren nach Anspruch 1, wobei die Gelierlösung im Schritt (b) bei 35-45 °C vorliegt.

3. Verfahren nach Anspruch 1, wobei die Vernetzungslösung im Schritt (c) bei 19 bis 23 °C vorliegt.

4. Verfahren nach Anspruch 1, das weiter den Schritt des Verpackens und dann Kühlens des Verbands auf etwa 5 °C umfasst.

5. Verfahren nach Anspruch 1, wobei die PVA-Gelierlösung 6 bis 10 % PVA m/V, 1-10 % eines Weichmachers m/V, 2 bis 8 % Aloe Vera m/V und 1-7 % eines Mittels umfasst, das aus der Gruppe ausgewählt ist, die aus Folgenden besteht: Aloe Vera, Paraben, Arnika, BHT, alpha-Tocopherol, Natriumdiacetat, Cineol, Menthol, Noni-Extrakt, Campher, Weißtannenöl, Immortelle, Betulin-alba-Terpenoiden, Lavendelöl-Derivaten, Zimtöl, Zinkascorbat-Dihydrat oder Zinkderivate, Niacin, alpha-Terpineol und Kombinationen davon.

6. Verfahren nach Anspruch 5, wobei der Weichmacher aus der Gruppe ausgewählt ist, die aus Propylenglycol, Polypropylenglycol, Ethylenglycol, Diethylenglycol, Triethylenglycol und Mischungen davon besteht.

7. Verfahren nach Anspruch 1, das weiter das Zugeben eines Zusatzstoffs umfasst, der aus Antioxidationsmitteln, antifungalen und antibakteriellen Mitteln ausgewählt ist.

8. Verfahren nach Anspruch 1, das weiter das Zugeben eines Therapeutikums umfasst.

9. Kompressionsverband, der durch ein Verfahren nach einem der vorstehenden Ansprüche erhalten werden kann.

## Revendications

1. Méthode de préparation d'un bandage compressif, comprenant :
i) une matrice fibreuse d'un réseau élastomère à cellules ouvertes, non tissé air-laid ou absorbant à base de pâte, à volume de vide supérieur ou égal à 70% ; et
ii) un réseau interpénétrant de PVA disposé au sein du réseau d'élastomère, où ladite méthode comprend :
(a) la préparation d'une solution gélifiante de PVA ;
(b) l'immersion de la matrice fibreuse dans la solution gélifiante ;
(c) l'addition d'une solution de réticulation comprenant du tétraborohydrate, du Borax ou décahydrate de bore ; et
(d) l'élimination de l'excès d'eau.

2. Méthode selon la revendication 1, dans laquelle la solution gélifiante se trouve à 35-45°C dans l'étape (b).

3. Méthode selon la revendication 1, dans laquelle la solution de réticulation se trouve à de 19 à 23°C dans l'étape (c).

4. Méthode selon la revendication 1, comprenant en outre l'étape d'emballage puis de refroidissement du bandage jusqu'à environ 5°C.

5. Méthode selon la revendication 1, dans laquelle la solution gélifiante de PVA comprend de 6 à 10% p/v de PVA, 1-10% p/v d'un plastifiant, de 2 à 8% p/v d'aloès et 1-7% d'un agent choisi dans le groupe constitué par l'aloès, un parabène, l'arnica, le BHT, l'alpha-tocophérol, le diacétate de sodium, le cinéol, le menthol, un extrait de Noni, le camphre, l'essence de sapin concolore, l'immortelle, les terpénoïdes de *Betula alba*, les dérivés d'essence de lavande, l'essence de cannelle, l'ascorbate de zinc dihydraté ou les dérivés de zinc, la niacine, l'alpha-terpinol, et des combinaisons de ceux-ci.

6. Méthode selon la revendication 5, dans laquelle le plastifiant est choisi dans le groupe constitué par le propylène glycol, le polypropylène glycol, l'éthylène glycol, le diéthylène glycol, le triéthylène glycol, et des mélanges de ceux-ci.

7. Méthode selon la revendication 1, comprenant en outre un additif choisi parmi les antioxydants, les agents antifongiques et antibactériens.

8. Méthode selon la revendication 1, comprenant en outre l'addition d'un agent thérapeutique.

9. Bandage compressif pouvant être obtenu par une méthode telle que définie selon l'une quelconque des revendications précédentes.
